Europäisches Patentamt

European Patent Office

Office européen des brevets

(11) Publication number: **0 415 666 A1**

## EUROPEAN PATENT APPLICATION

(21) Application number: 90309309.4

(51) Int. Cl.⁵: **C12N 5/00, A61K 35/16**

(22) Date of filing: 24.08.90

(30) Priority: 29.08.89 JP 222240/89

(43) Date of publication of application:
06.03.91 Bulletin 91/10

(84) Designated Contracting States:
AT BE CH DE DK ES FR GB GR IT LI LU NL SE

(71) Applicant: TAKEDA CHEMICAL INDUSTRIES, LTD.
3-6, Doshomachi 2-chome Chuo-ku
Osaka 541(JP)

Applicant: NIHON PHARMACEUTICAL Co., Ltd.
9-8, Higashikanda 1-chome, Chiyoda-ku
Tokyo 101(JP)

(72) Inventor: Sasai, Seijiro
4010-20, Murozumiyamane-cho
Hikari, Yamaguchi 743(JP)
Inventor: Tuchida, Tetsuo
2-48, Koyoen-Sannoucho
Nishinomiya, Hyogo 662(JP)
Inventor: Higashino, Kazuya
1-3, Inaba 3-chome
Higashiosaka, Osaka 578(JP)
Inventor: Kitauchi, Masahiro
317, 41-3, Higashikujo-nishikawabecho,
Minami-ku
Kyoto 601(JP)

(74) Representative: Lewin, John Harvey et al
ELKINGTON AND FIFE Beacon House 113
Kingsway
London WC2B 6PP(GB)

(54) Composition for human cell cultivation and use thereof.

(57) The present invention provides a composition for human cell cultivation that is substantially free of human cell growth inhibitors and microorganisms and that contains a human blood-derived growth and differentiation acceleration factor, which is excellent in terms of safety, involves only small lot difference and shows stable cytotoxicity irrespective of the blood group of the starting material blood.

## COMPOSITION FOR HUMAN CELL CULTIVATION AND USE THEREOF

Field of the invention

The present invention relates to a composition for human cell cultivation and a method of cultivation using it.

Background of the invention

Presently, malignant tumors are treated mainly by surgical therapy, chemotherapy and radiotherapy. Investigations have been made with the aim of improving therapeutic efficiency by appropriately combining these therapies. However, therapeutic results remain unsatisfactory. With this background, immunotherapy, aiming at an antitumor effect by potentiation of the immunoresistance of the host, has drawn much attention as a supplement to these three therapies.

A new anticancer therapy which, unlike conventional immunotherapy based on immunopotentiator administration, uses autologous lymphocytes with induced tumor cytotoxicities, has been under clinical trials by Rosenberg et al. at the US National Cancer Institute [Steven A. Rosenberg et al.: The New England Journal of Medicine, *313*, 1485-1492 (1985); Steven A. Rosenberg et al.: The New England Journal of Medicine, *316*,889-897 (1987)] following thorough investigation using experimental model systems with mice [Dononue J. H., Rosenstein M., Chang A. E., et al.: J. Immunol., *132*, 2123-2128 (1984); Eberlein T. J., Rosenstein M., Rosenberg S. A.: J. Exp. Med., *156*, 385-397 (1982)].

As reported by Rosenberg et al., this new immunotherapy is characterized by the final goal of tumor size reduction by inducing lymphokine-activated killer (LAK) cells possessing tumor cytotoxicities by cultivating peripheral blood lymphocytes of a cancer patient in the presence of a lymphokine such as interleukin 2 (hereinafter referred to as IL-2), a lymphocyte differentiation/induction/growth factor, *in vitro*, and then returning them to the patient. This mode of therapy is called adoptive immunotherapy. In Japan as well, intensive clinical trials have been made for these 2 or 3 years. Some case reports, from both Japan and abroad, state that this therapy was very effective on some different types of carcinoma. With the expectation to serve well as a new-generation anticancer therapy, it is now undergoing intensive investigations on a global scale.

Usually, LAK cells are induced from human lymphocytes using an RPMI-1640 basal medium for animal cell cultivation as a culture broth, in the presence of differentiation/induction/growth factor IL-2, human serum and antibiotics. The serum added to the culture broth is a 2 to 5% dilution of human serum of AB blood, and is used after inactivation by heating at 56°C for 30 minutes. The IL-2 added to the culture broth is used at concentration of about 100 to 1,000 JRU/ml. Cultivation is normally carried out at 37°C in the presence of 5% $CO_2$ by standing culture or rotation culture for 3 to 10 days.

Judging from information so far been reported from both Japan and abroad, it is evident that the effectiveness of the adoptive immunotherapy using LAK cells depends mainly upon the number of transferred lymphocytes for treatment, LAK cell activity and LAK cell administration site vicinity to tumor. Therefore, a key to successful application of the present therapy is maximization of the number of cultivated lymphocytes and improvement of LAK cell activating conditions, which require the supply of a considerable amount of human serum of the AB blood group. It is reported that such human serum is difficult to obtain. Also reported is the occurrence of hepatitis A in association with the use of serum obtained by blood donation for the present therapy [Steven A. Rosenberg et al.: The New England Journal of Medicine, *313*, 1485-1492 (1985)], posing a safety problem. The limited availability of safe serum is a deterrent to the performance of the present therapy.

The present inventors previously succeeded in preparing compositions for cell cultivation from bovine, equine, porcine and ovine serum and plasma, as disclosed in Japanese Unexamined Patent Publication Nos. 145088/1985 and 29968/1987. By making full use of know-how compiled through these investigations, the present inventors succeeded in producing a human blood derived composition for human cell cultivation with high compatibility with human cells.

Summary of the invention

The present invention comprises a composition for human cell cultivation that is substantially free of human cell growth inhibitors and microorganisms and which contains a human blood-derived growth and differentiation acceleration factor, a medium for human cell cultivation containing said composition and a basal medium, a method of human cell cultivation characterized by the use of said medium, and human cells cultivated by said method of cultivation. It is unique in that it significantly reduces the amount of human serum, from that required with conventional methods of cultivation, and that the risk of occurrence of viral infections due to direct addition of human serum to LAK cell cultivation system is avoidable. In addition, since the composition for human cell cultivation of the present invention is substantially free of anti-A and anti-B antibodies present in blood of groups A, B or O, the influence of such antibodies can be ignored, i.e., any blood group can serve as composition for human cell cultivation in the LAK cell cultivation system, offering a great advantage in view of the difficulty of obtainment of human serum of blood type AB as a starting material for a human-derived biological preparation. It should be noted that the proliferation rate and tumor cytotoxicity of LAK cells induced by cultivation were both significantly higher than those obtained by the conventional method of cultivation using an RPMI-1640 medium formulated with human serum of the blood group AB. Also, the composition for human cell cultivation of the present invention showed only a few differences among lots. It oan therefore be said that the present invention has made it possible to develop a better method of cultivation.


Brief description of the drawings

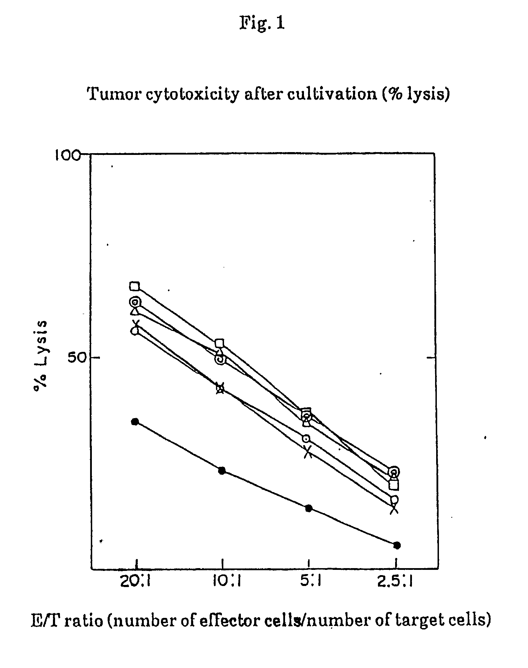

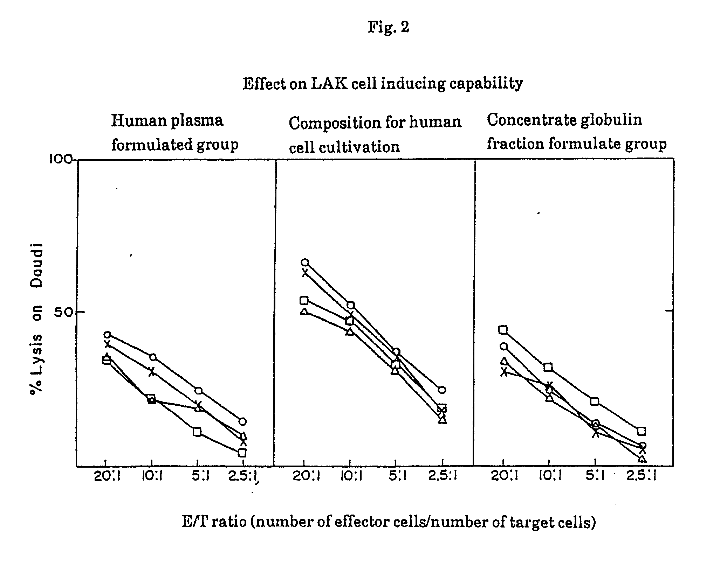

Figs. 1 and 2 show tumor cell cytotoxicity (% lysis) [effect on LAK cell inducing capability] in each cultivation system, obtained in Examples 2 and 3, respectively.

Detailed description of the preferred embodiments

The process for producing the composition for human cell cultivation of the present invention from the starting material human blood is as follows.

When using plasma as a starting material, it is preferable to proceed with a supernatant obtained by adding an anticoagulant (e.g. sodium citrate) to collected blood, centrifuging this mixture, adding a solution of calcium chloride to the resulting supernatant to reach a final concentration of about 20 mM to coagulate the fibrin, and then recentrifuging the mixture.

Here, a microorganism means a human-derived contaminant microorganism or a microorganism which may become a contaminant after blood collection, including viruses and mycoplasmas, which cannot be removed by ordinary sterilization using membrane filters. These microorganisms are inactivated as follows. An inactivator is used for the purpose of inactivation. It is preferable that the treatment be carried out by adding to serum or plasma an inactivator which has strong inactivating power and which does not significantly affect the human cell differentiation and growth accleration factor present in serum or plasma.

Examples of effective inactivators include $C_{2-4}$ alkenyl oxides such as ethylene oxide and propylene oxide and dialdehydes such as glyoxal and glutaraldehyde, of which ethylene oxide is particularly excellent from the viewpoint of inactivating power and the degree of influence on the differentiation and growth factor, with preference given to liquid ethylene oxide.

When using liquid ethylene oxide, the amount added is normally 0.1 to 5% by volume, preferably 0.3 to 2% by volume. Inactivation is carried out at 0 to 30°C, preferably at 10 to 30°C, for 1 to 7 days, preferably 1 to 3 days. Another inactivator can be used as above. In removing the inactivator added to inactivate the contaminant microorganisms, no special treatment is necessary; inactivator such as ethylene oxide can easily be removed by dialysis etc.

Human cell growth inhibitors are various substances which adversely affect or inhibit human cell growth, such as immunoglobulins present in serum or plasma, and are removed by the salting-out and desalting process of the present invention described below.

Differentiation and growth acceleration factors may be any factors which can accelerate growth and differentiation of human lymphocytes and, for example, include albumin, plasma proteins and various trace growth acceleration factors, etc. which are present in serum or plasma.

An example salting-out and desalting process is as follows:

For salting-out, inorganic and other salts are used. Examples of inorganic salts include ammonium salts (e.g. ammonium sulfate, ammonium chloride), a sodium salt (sodium chloride) and a potassium salt (potassium carbonate), with preference given to ammonium salts, particularly ammonium sulfate.

In the salting-out and desalting process, in accordance with an ordinary salting-out method, the starting material serum or plasma, with or without pretreatment by the contaminant microorganism inactivation process described above, is dissolved or suspended in a solvent (e.g., water, ethanol, hydrated ethanol). This solution or suspension is saturated with a salt until its concentration reaches a specified lower limit, followed by separating precipitate removal to yield a supernatant. This supernatant is saturated with a salt until its concentration reaches a specified upper limit, followed by separating precipitate collection to yield the desired fraction. This procedure is preferably carried out under nearly neutral conditions (pH 6 to 8).

More specifically, when using ammonium sulfate as such a salt, it is preferable to conduct salting-out at minimum concentration of not less than 40%, more preferably not less than 50%, and maximum concentration of not more than 80%, with particular preference given to the range of from 55% to 70%. When using another salt, salting-out is possible at given concentrations corresponding to the above-mentioned ammonium sulfate concentrations. Supernatant-precipitate separation is well achieved by centrifugation etc.

The obtained precipitate is dissolved in physiological saline etc. and then desalted by a method such as dialysis or ultrafiltration.

Dialysis can be carried out in accordance with a known method using, for example, a dialysis membrane. When conducting ultrafiltration, an ultrafilter membrane which passes substances of molecular weight not exceeding 1000, for instance, is used under increased pressure.

The obtained composition for human cell cultivation is prepared to yield a concentration of normally 20 to 80 mg/m ℓ with physiological saline etc. and filtered to remove germs through a membrane filter etc., after which it can be frozen or lyophilized for storage. In the production method for the composition for human cell cultivation of the present invention, either the contaminant microorganism inactivation process or the salting-out and desalting process may be conducted first.

The composition for human cell cultivation of the present invention is highly sterile and free of possibly contaminant filtrable microorganisms, such as viruses and mycoplasmas, and can be said to be suitable for cultivation of LAK cells to be administered to cancer patients as anticancer therapy. In addition, the composition for human cell cultivation of the present invention is capable of inducing a good cell growth accelerating effect and tumor cytotoxicity irrespective of the individual differences of the lymphocytes used.

The composition for human cell cultivation thus obtained is used to cultivate human cells (e.g. human lymphocytes) as follows. Cultivation is carried out in a mixture prepared by adding the composition for human cell cultivation alone or in combination with a trace growth promoter, such as insulin, transferrin, ethanolamine or sodium selenite, to a basal medium to yield a final concentration of about 0.1 to 10 mg/m ℓ, preferably about 0.5 to 5 mg/m ℓ, more preferably about 3 mg/m ℓ. Examples of the basal medium formulated with the composition for human cell cultivation include various basal media for animal cell cultivation, of which Daigo T medium (produced by Nippon Seiyaku; for composition, see Table 1) is preferred.

Table 1  Composition of Daigo T Medium mg/ℓ (15.5 g)

| | |
|---|---|
| Sodium hyposelenite | 0.0088 |
| HEPES | 4289.4 |
| Calcium chloride | 99.155 |
| Copper sulfate (pentahydrate) | 0.00125 |
| Ferrous sulfate (heptahydrate) | 0.417 |
| Potassium chloride | 277.0 |
| Potassium nitrate | 0.038 |
| Magnesium chloride (hexahydrate) | 61.051 |
| Magnesium sulfate | 48.835 |
| Sodium chloride | 6052.0 |
| Disodium hydrogenphosphate | 71.0 |
| Sodium dihydrogenphosphate | 62.5 |
| Zinc sulfate (heptahydrate) | 0.4315 |
| Glucose (anhydrous) | 3151.0 |
| Hypoxanthine | 2.040 |
| Linolic acid | 0.042 |
| DL-thioctic acid | 0.103 |
| Phenol red | 8.120 |
| Putrescine dihydrochloride | 0.0805 |
| Sodium pyruvate | 110.0 |
| Thymidine | 0.3635 |
| D-biotin | 0.01015 |
| Calcium D-pantothenate | 2.2385 |
| Coline chloride | 9.00 |
| Folic acid | 2.66 |
| Inositol | 12.60 |

| | |
|---|---|
| Amide nicotinate | 2.0185 |
| Pyridoxal hydrochloride | 2.000 |
| Pyridoxine hydrochloride | 0.031 |
| Riboflavin | 0.219 |
| Thiamine hydrochloride | 2.1685 |
| Cyanocobalamin | 0.6865 |
| L-alanine | 16.955 |
| L-arginine hydrochloride | 147.500 |
| L-asparagine (monohydrate) | 21.705 |
| L-aspartic acid | 21.655 |
| L-cysteine hydrochloride (monohydrate) | 17.560 |
| L-glutamic acid | 44.855 |
| L-glutamine | 365.0 |
| Glycine | 18.755 |
| L-histidine hydrochloride (monohydrate) | 31.480 |
| L-methionine | 17.240 |
| L-phenylalanine | 35.480 |
| L-proline | 37.265 |
| L-serine | 26.255 |
| L-threonine | 53.455 |
| L-tryptophan | 9.020 |
| L-thyrosine | 54.615 |
| L-valine | 52.855 |
| L-isoleucine | 54.470 |
| L-leucine | 59.060 |
| L-lysine hydrochloride | 91.270 |
| L-cystine dihydrochloride | 45.620 |

The composition for human cell cultivation of the present invention can be used as a composition for human lymphocyte cultivation for adoptive immunotherapy in an LAK cell cultivation system as described above. The human lymphocytes to be cultivated may be those derived from a cancer patient who is to be a subject of immunotherapy or those of a human who has the same major histocompatibility antigen as that of the cancer patient. Also, the LAK cell cultivation system may be formulated with PHA (phytohemagglutinin), concanavalin A, etc., in addition to a lymphokine (e.g. IL-2). The composition for human cell cultivation of the present invention can also be used for the cultivation of human lymphocytes such as TIL (tumor infiltrating lymphocyte), CTL (cytotoxic T lymphocyte) and A-LAK (adherent LAK), as well as LAK. It can also be used as a composition for human cell cultivation in cultivating human cells for the purposes of developing a lymphocyte bank (e.g., lymphocytes are collected and stored when the donor is young, and the stored lymphocytes are proliferated and returned to the donor's body when lymphocyte function has deteriorated when the donor becomes old), bone marrow transplantation, artificial tissue (e.g. artificial skin) grafting, encapsuled cell transfer (e.g., cells from a normal human are cultivated, encapsuled and transferred into the body of a diabetic), gene therapy (e.g., cells introduced with a normal gene are cultivated and transferred to a hereditary disease patient) and fetal tissue transfer.

The composition for human cell cultivation of the present invention is excellent in terms of safety since it is free of infectious microorganisms. Also, it involves only small lot difference and shows stable cytotoxicity irrespective of the blood group of the starting material blood. Therefore, it is advantageous over serum, and poses no problem as to quantitative availability. In addition, it exhibits an excellent tumor cytotoxicity inducing capability on human lymphocytes in the presence of IL-2, indicating potential for use as a substitute for human serum of blood type AB in adoptive immunotherapy.

## Examples

The present invention is hereinafter described in more detail by means of the following examples, but the invention is by no means limited by them.

In the present specification, when expressing IL-2 activity simply in U (unit), 1 U is equivalent to 383 JRU (Japanese reference unit).

Example 1

(Production of composition for human cell cultivation)

A composition for human cell cultivation was produced as follows. To the starting material frozen human plasma of the blood group AB, A, B or O (prepared by adding sodium citrate to blood by a standard method immediately after its collection, centrifuging this mixture and storing the resulting supernatant under freezing conditions), calcium chloride was added to reach a final concentration of 20 mM, followed by reaction in a water bath at 37°C for 2 hours with gentle shaking to coagulate the fibrin, which was centrifuged to yield a supernatant. Then, 0.75% (v/v) liquid ethylene oxide was added, and the mixture was kept standing at 25°C for 48 hours, followed by sterilization against contaminant microorganisms. Then, the salting-out fraction separated by saturation with 55 to 70% ammonium sulfate was collected and dissolved in physiological saline and dialyzed against physiological saline. Each dialyzate was filtered through a membrane filter (Milex GV, 0.22 μm, produced by Millipore Co.) to remove germs to yield a composition for human cell cultivation. The protein concentration of each obtained composition is as shown in Table 2.

Table 2

| Protein Concentration of Human Cell Cultivation Compositions Derived from Plasma of Various Blood Groups (mg/m$\ell$) | | | | |
|---|---|---|---|---|
| Human plasma blood group | A | B | O | AB |
| Protein concentration | 53.1 | 53.1 | 46.8 | 42.6 |

Example 2

Tumor cytotoxicity of human lymphocytes (1)

Using a medium prepared by adding to Daigo T medium human insulin (2 $\mu$g/m$\ell$, Shionogi), human transferrin (2 $\mu$g/m$\ell$, Sigma), ethanolamine (0.122 $\mu$g/m$\ell$, Wako Pure Chemical) and the composition for human cell cultivation produced in accordance with Example 1 (3 mg/m$\ell$), the tumor cytotoxicity of human lymphocytes was determined. As controls were used a medium prepared in the same manner as above but with human serum albumin (3 mg/m$\ell$, Nippon Seiyaku) added in place of the composition for human cell cultivation (3 mg/m$\ell$), and another medium prepared by adding 5% human serum of blood group AB to RPMI-1640 medium. Lymphocytes were separated from normal human peripheral blood by the specific gravity centrifugal method using a lymphocyte separation medium (LSM, Organon Technica). In the cytotoxicity test, Daudi lymphoma cells [Eva Klein et al.: Cancer Research, *28*, 1300 (1968)], a cultured human cancer cell line were used. Lymphocytes were suspended in each of the media described above as formulated with 3 U/m$\ell$ gene recombinant human IL-2. This suspension was seeded on a 24-well cell cultivation plate (25820, Corning) at 2 × $10^6$ lymphocytes per well, followed by incubation at 37°C in the presence of 5% $CO_2$ in a carbon dioxide incubator (LNA 122D, Tabai Espec) for 1 week.

After 1-week incubation, lymphocytes were harvested from the 24-well plate, and viable cell count was taken by the dye exclusion method using trypan blue. These viable cells, as effector cells for the cytotoxicity test, were added to a 96-well (round-based) cell cultivation plate (25850, Corning) in 4 amounts: 2 × $10^5$, 1 × $10^5$, 0.5 × $10^5$ and 0.25 × $10^5$ cells per well, in triplicate for each amount: Cytotoxicity was expressed as the average of 3 wells for each amount. As target cells, Daudi lymphoma cells isotope-labeled with sodium chromate $Na_2{}^{51}CrO_4$ (NEZ030S20, New England Nuclear) were added to the 96-well plate containing the effector cells, followed by incubation for 4 hours, and then a $^{51}Cr$ release test was conducted by a standard method. Cytotoxicity is expressed in both % lysis and lytic unit$_{50}$ (LU$_{50}$) (the number of effector cells required to cause 50% lysis was taken as 1 LU$_{50}$) per $10^6$ effector cells. As seen in Fig. 1, irrespective of blood group (AB: ○ - ○, A: □ - □,

B: △ - △, O: ◎ - ◎),

the composition for human cell cultivation showed stronger cytotoxicity in comparison with the ordinary cultivation system comprising RPMI-1640 + 5% serum of the blood group AB (● - ●). There was no significant activity difference among the compositions for human cell cultivation in association with blood group. When purified human serum albumin was used as a control (x - x), similar activity was obtained. It should be noted, however, that cell proliferation rate (% recovery) showed a noticeable decline in the group formulated with purified human serum albumin, as shown in Table 3, with significant differences in total LU (total number of recovered cells per well of the 24-well plate upon LAK cell induction converted from LU$_{50}$, calculated as LU$_{50}$ × % recovery × 1/100 × 2) in comparison with the groups formulated with a composition for human cell cultivation, in which cell proliferation rate was uniform in the range of from about 90 to 110%.

As is evident from Table 3, in terms of total LU as sum of cell proliferation and differentiation, the groups formulated with a composition for human cell cultivation greatly surpass the other cultivation systems.

### Table 3   Lymphocyte Proliferation Rate and Tumor Cytotoxicity after Cultivation (LU$_{50}$)

| Growing system \ Proliferation rate/ activity | LU$_{50}$ (Lytic Unit$_{50}$) | % Recovery | Total LU (LU$_{50}$ × % Recovery × 1/ 100 × 2) |
|---|---|---|---|
| ● - ● (5% serum of the blood group AB + RPMI-1640) | 3.1 | 168 | 10 |
| ○ - ○ (Medium formulated with a composition for human cell cultivation derived from blood AB | 15 | 101 | 30 |
| □ - □ (Medium formulated with a composition for human cell cultivation derived from blood A | 22 | 106 | 47 |
| △ - △ (Medium formulated with a composition for human cell cultivation derived from blood B | 19 | 106 | 40 |
| ◎ - ◎ (Medium formulated with a composition for human cell cultivation derived from blood O | 20 | 91 | 36 |
| × - × (Medium formulated with human serum albumin) | 14 | 46 | 13 |

Example 3

Tumor cytotoxicity of human lymphocytes (2)

Plasma (5%, about 3 mg/m$\ell$ as protein content) before use for the production of the composition for human cell cultivation and the concentrate globulin fraction obtained by 0 to 55% ammonium sulfate salting-out in the production process for the composition for human cell cultivation (about 3 mg/mℓ as protein content) were examined for inhibitor removing effect for all blood groups (A: O - O, B: Δ - Δ, O: □ - □, AB: x - x), in the same manner as in Example 2.

As is evident from Fig. 2, the groups formulated with a composition for human cell cultivation all showed stronger cytotoxicity in comparison with the groups formulated with either human plasma or the concentrate globulin fraction, irrespective of blood group. This suggests the potential of production of a composition for human cell cultivation with stable activity irrespective of blood group.

## Claims

1. A composition for human cell cultivation that is substantially free of human cell growth inhibitors and microorganisms and that contains a human blood-derived growth and differentiation acceleration factor.

2. A composition according to claim 1, wherein the human blood is human plasma.

3. A composition according to claim 2, wherein the human plasma is that treated with a solution of calcium chloride.

4. A composition according to claim 1, wherein the composition is soluble at the concentration of not less than 40% saturation and insoluble at the concentration of not higher than 80% saturation of inorganic salt solution in terms of ammonium sulfate solution.

5. A composition according to claim 4, wherein the composition is soluble at the concentration of not less than 55% saturation and insoluble at the concentration of not higher than 70% saturation.

6 A composition according to claim 1, which is prepared by subjecting a human blood to treatments comprising a step of inactivating microorganisms and a step of salting out and desalting to obtain a fraction containing a human blood-derived growth and differentiation acceleration factor and substantially free of human cell growth inhibitors.

7. A composition according to claim 6, wherein the step of inactivating microorganisms is conducted by contacting the human blood with an inactivating agent of $C_{2-4}$ alkenyl oxide or dialdehyde.

8. A composition according to claim 7, wherein $C_{2-4}$ alkenyl oxide is ethylene oxide.

9. A composition according to claim 8, wherein the ethylene oxide is in a form of liquid.

10. A composition according to claim 6, wherein the step of inactivating microorganisms is conducted by contacting the human plasma with liquid ethylene oxide at the concentration of 0.1 to 5% by volume at a temperature of 0° to 30°C for 1 to 7 days.

11. A composition according to claim 6, wherein the salting out is conducted at a lower limit concentration of not less than 40% saturation and at an upper limit concentration of not higher than 80% saturation of inorganic salt in terms of the ammonium sulfate.

12. A composition according to claim 11, wherein the lower limit concentration is not less than 55% saturation and the upper limit concentration is not higher than 70% saturation.

13. A composition according to claim 11, wherein the inorganic salt is an ammonium salt.

14. A composition according to claim 13, wherein the ammonium salt is ammonium sulfate.

15. A composition according to claim 11, wherein the salting out is conducted in an aqueous solution.

16. A composition according to claim 6, wherein the desalting is conducted by dialysis.

17. A composition according to claim 6, wherein the steps salting out and desalting are conducted after the step of inactivating microorganisms.

18. A composition according to claim 1, wherein the human cell is human lymphocytes.

19. A composition according to claim 1 characterized by use for human lymphocyte cultivation in adoptive immunotherapy.

20. A medium for human cell cultivation which contains the composition according to claim 1 together with a basal medium.

21. A medium according to claim 20, wherein the composition is in a concentration of 0.1 to 10 mg/ml.

22. A medium according to claim 20, which further contains trace growth promoters.

23. A medium according to claim 20, which further contains a lymphokine.

24. A method of cultivating human cells which comprises cultivating human cells in the medium according to claim 20.

25. A method according to claim 24, wherein the human cells are human lymphocytes.

26. A method of human lymphocyte cultivation for adoptive immunotherapy characterized in that human lymphocytes are cultivated in a medium prepared by adding a composition according to claim 19 and a lymphokine to a basal medium to grow and activate the human lymphocytes.

27. Human cells cultivated by a method of cultivation according to claim 24.

Fig. 1

Tumor cytotoxicity after cultivation (% lysis)

E/T ratio (number of effector cells/number of target cells)

## Fig. 2

### Effect on LAK cell inducing capability

Human plasma formulated group | Composition for human cell cultivation | Concentrate globulin fraction formulate group

% Lysis on Daudi

E/T ratio (number of effector cells/number of target cells)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WORLD PATENT INDEX (LATEST), accession no. 87-076442, week 11, Derwent Publications Ltd, London, GB; & JP-A-62 029 968 (AGENCY OF IND. SCI. TECH.) | 1-27 | C 12 N 5/00 <br> A 61 K 35/16 |

TECHNICAL FIELDS SEARCHED (Int. Cl.5)

A 61 K

The present search report has been drawn up for all claims

| Place of search | Date of completion of the search | Examiner |
|---|---|---|
| THE HAGUE | 27-11-1990 | TURMO Y BLANCO C.E. |